(19) Europäisches Patentamt
European Patent Office
Office européen des brevets

(11) Numéro de publication: **0 082 818**
**B1**

# (12) FASCICULE DE BREVET EUROPEEN

(45) Date de publication du fascicule du brevet: **23.10.85**

(51) Int. Cl.⁴: **C 07 G 17/00**, C 07 H 15/04, A 23 J 7/00

(21) Numéro de dépôt: **82810542.9**

(22) Date de dépôt: **15.12.82**

## (54) Procédé pour l'obtention de lipides d'origine humaine et du lacto-N-norhexaosyl ceramide.

(30) Priorité: **17.12.81 CH 8056/81**

(43) Date de publication de la demande: **29.06.83 Bulletin 83/26**

(45) Mention de la délivrance du brevet: **23.10.85 Bulletin 85/43**

(84) Etats contractants désignés: **AT BE DE FR GB IT LU NL SE**

(56) Documents cités:
**WO - A - 81/03175**
**FR - A - 1 577 393**
**FR - A - 2 194 413**

**BIOLOGICAL ABSTRACTS, vol. 60, no. 7, juillet 1975, no. 37678, Philadelphia, Pennsylvania (USA); P. HANFLAND et al.: "Quantitative isolation and purification of blood group-active glycosphingolipids from human B erythrocytes".**
**CHEMICAL ABSTRACTS, vol. 83, no. 9, 1 septembre 1975, page 268, no. 74004q, Columbus Ohio (USA); E.L. SMITH et al.: "Identification of a novel heptaglycosylceramide with two fucose residues and a terminal hexosamine".**
**CHEMICAL ABSTRACTS, vol. 80, no. 23, 10 juin 1974, page 326, no. 131485m, Columbus Ohio (USA); B.L.**

(73) Titulaire: **Laboratoire Lucchini S.A., 20, rue de la Coulouvrenière, CH-1204 Genève (CH)**

(72) Inventeur: **Martin, Michel, rue Crespin, 16, CH-1206 Genève (CH)**

(74) Mandataire: **Roth, Pierre et al, PIERRE ARDIN & CIE 22, rue du Mont-Blanc, CH-1201 Genève (CH)**

(56) Documents cités: (suite)
**SLOMIANY et al.: "Blood group A active ceramide hexasaccharide lacking N-acetylglucosamine isolated from hog stomach mucosa".**
**BIOLOGICAL ABSTRACTS, vol. 67, no. 8, août 1979, no. 47856, Philadelphia, Pennsylvania (USA); P. HANFLAND et al.: "Isolation and purification of Lewis blood-group active glycosphingolipds from the plasma of human O Le individuals"**
**BIOLOGICAL ABSTRACTS, vol. 58, no. 11, novembre 1974, no. 47383, Philadelphia, Pennsylvania (USA); H. WIEGANDT: "Monosialo-lactotsohexaosylceramide: A ganglioside from human spleen".**
**BIOLOGICAL ABSTRACTS, vol. 71, no. 12, decembre 1981, no. 81647, Philadelphia Pennsylvania (USA); T. ITOH et al.: "Isolation and characterization of novel monosialosylpentahexosyl ceramide from tay-sachs brain".**
**R. RIEMSCHNEIDER & M.Z. ABEDIN, Die Angewandte Makromolekulare Chemie, 82 (1972), 171-186, No. 1276**

EP 0 082 818 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention a pour objet un procédé pour l'obtention du lacto-N-norhexaosyl céramide par extraction à partir d'un tissu placentaire humain riche en ce ganglioside.

Par la publication de R. Riemschneider et M. Z. Abedin dans »Die Angewandte Makromolekulare Chemie 82 (1979) 171—186 (Nr. 1276), on connait déjà un procédé pour extraire de tissus placentaires des lipides ou phospholipides par prétraitement à l'éther suivi d'un lavage des résidus avec de l'hydroxyde ou de l'acétate de sodium.

On peut obtenir du lacto-N-norhexaosyl céramide par extraction à partir d'un tissu placentaire humain par un procédé qui consiste à extraire en un premier temps et de façon connue les gangliosides bruts au moyen d'un mélange de solvant polaire, tel que le chloroforme, le chlorure de méthylène, l'éther éthylique ou l'éther isopropylique, et de solvant apolaire, tel que le méthanol, éthanol ou isopropanol, en présence d'eau, à raison de 28,1% de solvant polaire, 56,3% de solvant apolaire et de 15,6% d'eau, en volume, à séparer les gangliosides bruts de la couche aqueuse par évaporation du solvant, à purifier les gangliosides bruts par saponification douce dans un solvant apolaire en présence d'eau, dialyse des gangliosides dans la solution restante et évaporation de cette dernière.

Toutefois, on ne connait pas jusqu'à présent de procédé permettant de séparer industriellement, à partir des gangliosides bruts, des gangliosides purifiés, tels que les esters sialyliques de l'hématoside, du paragloboside et du lacto-N-norhexaosyl céramide.

Le procédé selon l'invention remédie à cet inconvénient et permet d'obtenir du lacto-N-norhexaosyl céramide et à partir de gangliosides bruts isolés de tissus placentaires humains repris dans un mélange de solvant comprenant deux parties de solvant polaire pour une partie de solvant apolaire en volume qu'on filtre. Il est caractérisé en ce qu'on précipite les gangliosides par d'acétate d'éthyle, les impuretés restant dans le solvant, tandis que les gangliosides purifiés, contenant en proportions sensiblement égales des esthers sialyliques de l'hématoside, du paragloboside et du lacto-N-norhexaosyl céramide, sont séparés par filtration ou essorage.

On peut préparer le lacto-N-norhexaosyl céramide à partir de ces gangliosides purifiés par une hydrolyse ménagée, par exemple dans un mélange méthanol/eau (80 : 20, volume par volume). Dans ce cas, on ajoute du chloroforme et de l'eau afin d'amener les proportions de constituants du solvant à 20 parties de chloroforme, 10 parties de méthanol et 6 parties d'eau. La phase aqueuse est séparée par décantation ou centrifugation. Après une seconde extraction, on réunit les phases aqueuses et les évapore pour obtenir le lacto-N-norhexaosyl céramide pratiquement pur.

Du fait que, selon l'invention, il est possible d'extraire industriellement en quantités suffisantes à partir de tissu placentaire riche en sialyl-lacto-N-norhexaosyl céramide, dont la copule neutre

(Gal$\beta$1 →4 GlcNAc$\beta$1 →3 Gal$\beta$1 →4 GlcNAc$\beta$1 →3 Gal$\beta$1 →4 Glc →céramide)

présente une forte activité antigénique i, du lacto-N-norhexaosyl céramide pratiquement pur, on peut l'utiliser dans le diagnostic et le traitement de certaines maladies autoimmunes ou dans certaines tumeurs, du fait de son accessibilité suffisante.

Ainsi il devient par exemple possible, par l'utilisation du lacto-N-norhexaosyl céramide, copule neutre, de diagnostiquer et de traiter le »cold agglutinine disease« (voir Watanabe K., Powell M. E., Hakomori S., Childs R. A., Feizi T., Biochem. Biophys. Res. Comm. 81, 1286—1293, 1978) ou certaines tumeurs (voir Surani M. A. H. Cell 18, 217—227, 1979). Comme matière première, dans le procédé selon l'invention, ont peut utiliser des gangliosides d'origine placentaire, extraits de placenta frais ou de résidus résultant de la fabrication de $\gamma$-globulines, d'extraits ou d'enzymes placentaires.

Les gangliosides d'origine placentaire humaine (hématoside-GM3, sialyl-i) contiennent exclusivement de l'acide N-acétyl-neuraminique par opposition aux gangliosides provenant de tissus d'autres mammifères qui contiennent des quantités variables suivant l'espèce d'acide N-glycolyl-neuraminique. En outre, les lipides résiduels obtenus dans cette préparation peuvent constituer une source appréciable de phospholipides d'origine humaine, dont l'utilisation en thérapeutique est de plus en plus importante comme véhicule de médicaments spécifiques, ou de lipides utilisables en thérapeutique ou en cosmétique.

### Préparation de la matière première

1 kg de placenta ou de résidu de placenta est broyé dans 7,5 l de chloroforme-méthanol (2 : 1, v/v), le tout est laissé en contact 2 heures à la température ambiante, puis filtré. L'extrait est conservé et le résidu solide est repris dans 7,5 l du mélange chloroforme-méthanol (1 : 1, v/v), rebroyé et laissé 2 heures à la température ambiante, puis filtré. L'extrait obtenu, mélangé à l'extrait précédent, est évaporé sous vide, fournissant ainsi de 30 à 40 g de résidu sec. Ce résidu est redissous dans 2 l de chloroforme-méthanol (2 : 1, v/v) auquel on ajoute 400 ml d'eau bidistillée, on agite et l'on sépare les 2 phases formées par centrifugation ou décantation. La phase supérieure aqueuse (extrait A) est conservée. La phase inférieure (chloroformique) est additionnée de 650 ml de méthanol, ce qui donne après

agitation une solution homogène à laquelle on ajoute 400 ml d'eau bidistillée. Après agitation, on sépare comme précédemment les 2 phases. La phase supérieure aqueuse est mélangée avec le premier extrait A et le tout est évaporé après addition de Toluène ou de butanol, comme anti-mousse. Le résidu obtenu de 10 à 20 g contient la majeure partie des gangliosides du placenta. La phase chloroformique, après évaporation, fournit de 20 à 30 g de lipides totaux contenant les lipides neutres, les phospholipides et les glycolipides neutres du placenta.

Variante de préparation

1 kg de résidu de placenta est broyé avec 2 l d'eau distillée et 7,2 l de méthanol; on ajoute ensuite 3,6 l de chloroforme. On agite 30 minutes, puis on filtre. Le résidu est réextrait, après réhomogénéisation dans 1,3 l d'eau, par 5,3 l du mélange chloroforme-méthanol (1 : 2, v/v); on filtre et les 2 extraits sont mélangés. On y ajoute alors 3,5 l d'eau bidistillée, on agite et on sépare les 2 phases par centrifugation ou décantation. La phase supérieure aqueuse, contenant la majeure partie des gangliosides, est évaporée sous vide en présence de butanol comme anti-mousse. Le résidu sec obtenu de la phase chloroformique contient l'ensemble des autres lipides.

Autre variante de préparation

1 kg de résidu de placenta broyé est extrait 2 fois à froid par 5 volumes d'acétone, pour éliminer les lipides neutres. Le tissu dégraissé est alors extrait à reflux par de l'éthanol. L'extrait éthanolique est soit concentré et les glycolipides mis à précipiter par refroidissement à —4° C pendant 24 heures, soit évaporé à sec, puis redissous dans le mélange chloroforme-methanol (2 : 1, v/v) et les gangliosides extraits par addition de 0,2 volume d'eau comme décrit à l'exemple 1

Afin de le purifier, l'extrait sec de ganglioside brut est dissous dans 200 ml de NaOH méthanolique 0,2 M et laissé 2 h à 37° C. Après neutralisation, la solution est évaporée à sec et le résidu repris dans 100 ml d'eau bidistillée et dialysée pendant 48 heures.

Exemple de procédé selon l'invention

La solution de la variante précédente est évaporée à sec et le résidu est redissout dans 20 ml de chloroforme-méthanol (2 : 1, v/v), on filtre pour éliminer l'insoluble et les gangliosides sont précipités par addition de 170 ml d'acétate d'éthyle. Le précipité d'environ 0,6 à 1 g contient la majeure partie des gangliosides.

La phase de saponification peut toutefois être abandonnée et la dialyse de l'extrait brut dissout dans l'eau peut être effectuée immédiatement. La précipitation par l'acétate d'éthyle de cet extrait entraîne une plus forte proportion de sphingomyéline dans le précipité

Les gangliosides peuvent être purifiés à partir de ce précipité par dissolution dans 100 ml de mélange chloroforme-méthanol (2 : 1, v/v) et extraction à l'eau comme précédemment décrit. On obtient ainsi un extrait aqueux de 0,1 à 0,2 g de gangliosides purifiés constitués principalement de $GM_3$ (Hématoside), de Sialyl-paragloboside et de Sialyl-lacto-N-norhexaosyl céramide, en proportions à peu près équilibrées.

On peut aussi, à partir du précipité de gangliosides bruts ou à partir des gangliosides purifiés, préparer le lacto-N-norhexaosyl céramide par hydrolyse ménagée.

Les gangliosides purifiés sont dissous dans 20 ml d'HCl 0,2 N dans le mélange méthanol-eau (80 : 20, v/v) et laissé 1 heure à 80°C. On ajoute du chloroforme et de l'eau pour obtenir un mélange chloroforme-méthanoleau 20 : 10 : 6 (v/v). La phase aqueuse est séparée par décantation ou par centrifugation. Une deuxième extraction est effectuée et les phases aqueuses réunies sont évaporées et fournissent de 50 à 150 mg de Lacto-N-norhexaosyl céramide contenant des traces de paragloboside.

Cette hydrolyse peut être obtenue par chauffage à 100° C pendant 1 heure des gangliosides dissous dans de l'eau bidistillée additionnée de 1% d'acide acétique. La purification se fait aussi par partage entre une phase chloroformique et une phase aqueuse comme précédemment décrit.

La pureté des composés obtenus peut être vérifiée par chromatographie sur plaque de gel de silice H dans le solvant chloroforme-méthanol-eau (60 : 35 : 8, v/v) et révélation à l'orcinol-acide sulfurique. Le glycolipide i présente un Rf caractéristique. D'autre part, son activité antigénique i peut être mesurée par inhibition de l'hémaglutination en utilisant des sérums standards anti-i.

Les gangliosides présentent aussi un comportement chromatographique spécifique. Leur identité peut être confirmée par hydrolyse à la neuraminidase. Dans le cas du Sialyl-lacto-N-norhexaosyl céramide, l'apparition de l'activité antigénique i peut être mise en évidence après action de la neuraminidase.

De préférence les gangliosides sont chromatographiés en bande sur une plaque préparative de gel de silice H, puis on révèle sur une partie de la plaque et on élue chacune des fractions de gangliosides au mélange chloroforme-méthanol-eau (1 : 2 : 1,4, v/v). On évapore et on reprend au tampon acétate pH 5,8 contenant 25 U de neuraminidase et 0,1% de $CaCl_2$. On laisse 24 h à 37° C; on évapore après addition de méthanol, reprend le résidu par un mélange de chloroforme-méthanol-eau (60 : 30 : 4,5) et

on élimine les sels minéraux par chromatographie sur Sephadex® G. 25. La chromatographie de l'éluat sur plaque de gel de silice comme précédemment montre présence de CDH provenant du $GM_3$ de paragloboside (PG) provenant du Sialyl-paragloboside (SPG) et du glycolipide i provenant de l'hydrolyse du Sialyl-i.

Les phases chloroformiques provenant de l'extraction des gangliosides contiennent une forte proportion de sphingomyéline (SPH) et de phosphatidylcholine (PC) qu'il est possible de purifier.

**Revendications**

1. Procédé pour l'obtention du lacto-N-norhexaosyl céramide, de formule:

Galβ1 →4 GlcNAcβ1 →3 Galβ1 →4 GlcNAcβ1 →3 Galβ1 →4 Glc →Cer,

à partir des gangliosides bruts isolés de tissu placentaire humain, repris dans un mélange de solvants comprenant deux parties de solvant polaire et une partie de solvant apolaire en volume, qu'on filtre, caractérisé en ce qu'on précipite les gangliosides par adjonction d'acétate d'éthyle, les impuretés restant dans le solvant, tandis que les gangliosides purifiés, contenant en proportions sensiblement égales des esters sialyliques de l'hématoside, du paragloboside et du lacto-N-norhexaosyl céramide, sont séparés par filtration ou essorage.

2. Procédé selon la revendication 1, caractérisé en ce qu'on purifie le mélange des esters sialyliques obtenus par filtration ou essorage par une hydrolyse ménagée dans le méthanol en présence d'eau, qu'on ajoute du chloroforme à la solution hydrolysée afin d'amener les proportions des constituants du solvant sensiblement à 20 parties de chloroforme, 10 parties de méthanol et 6 parties d'eau, qu'on sépare les deux couches ainsi formées, qu'on procède à une seconde extraction et qu'on réunit les phases aqueuses qu'on évapore pour obtenir le lacto-N-norhexaosyl céramide pratiquement pur.

3. Procédé selon la revendication 1, caractérisé en ce qu'on ajoute l'acétate d'éthyle à raison de sensiblement 8,5 fois le volume du mélange de solvants.

**Patentansprüche**

1. Verfahren zur Gewinnung von Lacto-N-nor-hexaosyl-ceramid der Formel

Galβ1 →4 GlcNAcβ1 →3 Galβ1 → 4 GlcNAcβ1 →3 Galβ1 →4 Glc →Cer,

ausgehend von rohen Gangliosiden, isoliert aus menschlichem Plazentagewebe, aufgenommen in einem Gemisch von Lösungsmitteln aus zwei Vol.-Teilen eines polaren Lösungsmittels und einem Vol.-Teil eines apolaren Lösungsmittels, welches man filtriert, dadurch gekennzeichnet, daß man die Ganglioside durch Zusatz von Äthylacetat ausfällt, wobei die Verunreinigungen in dem Lösungsmittel verbleiben, während die gereinigten Ganglioside, welche in annähernd gleichen Teilen Sialylester von Hämatosid, Paraglobosid und Lacto-N-norhexaosyl-ceramid enthalten, durch Filtrieren oder Absaugen abgetrennt werden.

2. Verfahren nach Anspruch 1, dadurch gekennzeichnet, daß man das durch Filtrieren oder Absaugen erhaltene Gemisch von Sialylestern durch eine in Methanol in Gegenwart von Wasser durchgeführte Hydrolyse reinigt, daß man der hydrolysierten Lösung Chloroform zusetzt, um die Anteile der Lösungsmittelbestandteile auf annähernd 20 Teile Chloroform, 10 Teile Methanol und 6 Teile Wasser zu bringen, daß man die so gebildeten zwei Schichten trennt, daß man eine zweite Extraktion vornimmt und daß man die wässerigen Phasen vereinigt, welche man eindampft, um das praktisch reine Lacto-N-norhexaosyl-ceramid zu erhalten.

3. Verfahren nach Anspruch 1, dadurch gekennzeichnet, daß man das Äthylacetat in einer Menge von annähernd dem 8,5fachen Volumen des Gemisches der Lösungsmittel zusetzt.

**Claims**

1. Process for obtaining lacto-N-norhexaosyl ceramide, of formula:

Galβ1 →4 GlcNAcβ1 →3 Galβ1 →4 GlcNAcβ1 →3 Galβ1 →4 Glc →Cer,

from isolated raw gangliosides of human placental tissue, dissolved in a mixture of solvents comprising two parts polar solvent and one part apolar solvent by volume, that is filtered, characterized in that the gangliosides are precipitated by addition of ethyl acetate, the impurities remaining in the solvent, while the purified gangliosides, containing in approximately equal proportions, sialyl esters of hematoside, paragloboside and lacto-N-norhexaosyl ceramide, are separated by filtration or draining.

2. Process according to claim 1, characterized in that the mixture of sialyl esters obtained by filtration or draining are purified by cautions hydrolysis made in methanol in the presence of water, that chloroform is added to the hydrolyzed solution to bring the proportions of the constituents of the solvent approximately to 20 parts chloroform, 10 parts methanol and 6 parts water, that the two layers thus formed are separated, that a second extraction is performed and that the aqueous phases are evaporated so as to obtain practically pure lacto-N-norhexaosyl ceramide.

3. Process according to claim 1, characterized in that the ethyl acetate is added at a rate of approximately 8.5 times the volume of the mixture of solvents.